# EUROPEAN PATENT SPECIFICATION

(11) **EP 2 198 263 B1**
(45) Date of publication and mention of the grant of the patent: **14.08.2019**
(21) Application number: 07818924.8
(22) Date of filing: 11.10.2007
(51) Int. Cl.: G01N 3/56, A47L 11/00, A47L 11/34, G01N 33/36

(54) **METHOD FOR TESTING THE IMPACT OF A CLEANING PROCESS ON A PROPERTY OF A FABRIC, AN APPARATUS FOR USE IN SUCH A METHOD, AND USE OF SAID METHOD OR APPARATUS FOR LABELING A FABRIC**
VERFAHREN ZUM PRÜFEN DER AUSWIRKUNG EINES REINIGUNGSVORGANGS AUF EINE EIGENSCHAFT EINES STOFFS, VORRICHTUNG ZUR VERWENDUNG BEI EINEM SOLCHEN VEFAHREN UND VERWENDUNG DES VERFAHRENS ODER DER VORRICHTUNG ZUM KENNZEICHNEN EINES STOFFS
PROCÉDÉ POUR TESTER L'IMPACT D'UN PROCESSUS DE NETTOYAGE SUR UNE PROPRIÉTÉ D'UN TISSU, APPAREIL À UTILISER DANS UN TEL PROCÉDÉ, ET UTILISATION DUDIT PROCÉDÉ OU DUDIT APPAREIL POUR L'ÉTIQUETAGE D'UN TISSU

(43) Date of publication of application: 23.06.2010
(73) Proprietor: Reutelingsperger, Christiaan M. H. G., 5944 BK Arcen (NL)
(72) Inventor: Reutelingsperger, Christiaan M. H. G., 5944 BK Arcen (NL)
(74) Representative: Ter Meer Steinmeister & Partner
(86) International application number: PCT/EP2007/008853
(87) International publication number: WO 2009/046741

(56) References cited:
- US-A- 2 966 790
- US-A- 5 522 251
- US-A- 5 563 329
- SCHIEFER H F: "Wear testing of carpets" U S BUR STANDARDS -- J RESEARCH; UNITED STATES BUREAU OF STANDARDS -- JOURNAL OF RESEARCH NOV 1942, vol. 29, no. 5, November 1942 (1942-11), pages 333-379, XP008086087

## Description

The present invention pertains to a method for testing an impact of a cleaning head constituted to execute a cleaning process involving mechanical load, on a property of a fabric. The invention also pertains to an apparatus constituted to apply said method and the use of said method or apparatus for providing fabrics with dedicated labels depicting their suitability for application of the cleaning head to clean the fabric.

Various apparatus are known to test the stability, in particular colour fastness and resistance to wear, of fabrics (i.e. a textile based material, wherein the textile is produced by knitting, weaving, felting, pressing or otherwise associating fibers). In particular such apparatus are known for fabrics that should withstand high mechanical load during their life-time, such as carpets and furniture (upholstery) fabrics. The so-called Crockmaster, commercially available from James H. Heal, Richmond Works, Halifax, West Yorkshire, England, is a tester designed for establishing the colour fastness of floor coverings, textile etc. to rubbing. The apparatus has a very simple design: a sample of a material to be tested is clamped in the apparatus and a rubbing finger, provided with a rubbing cloth, is translated under pressure over the sample for a number of times. When the test is completed, the cloth is inspected for colour change, which on its turn is an indication for the colour fastness of the sample. The Crockmaster is relatively cheap and therefore its use has become widespread. An important disadvantage of the Crockmaster is that the outcome of the test is in many cases not representative for the colour fastness of a carpet to a cleaning process using a cleaning head that contacts the carpet.

From US 5,522,251 assigned to Interface Inc., Atlanta, Georgia, USA, a system for simulating the effects of cleaning and maintenance on the appearance of floor coverings is known. This system comprises a turntable for securing samples of the covering to be examined, and a frame for securing wear inducing items as well as cleaning heads of cleaning devices. Although this system may be useful to get an indication for various types of floor coverings about their resistance against a combined load of a purely wear inducing item, in particular items provided with castors, and a cleaning head of a cleaning device, it appears to be not suitable to arrive at a good prediction of the resistance against change of one or more properties of a carpet when subjected to prolonged cleaning with a cleaning device that operates via a cleaning head that is pressed against the carpet under a mechanical load. The reason for this is not completely clear but may be related to the typical structure of carpet which is totally different from other floor coverings such as ceramic tiles, wood, stone, synthetic laminate, vinyl lino, linoleum etc. Carpet is a textile based floor covering. It usually consists of dyed pile yarns, a primary backing in which the yarns are sewn and a secondary backing that adds strength to the carpet. Typically an adhesive binds the primary and secondary backings. Over ninety percent of pile yarns today are made up of synthetic polymers, the rest of the yarns are wool and comprise the more expensive, woven carpet. Synthetics are plastics such as nylon, acrylics, polyester and polypropylene. Both the primary and secondary backing are largely made of woven or non-woven polypropylene, though some secondary backing may still be made of jute. It is important to note that because of the typical structure of carpet, it has very typical properties: it is non-homogenous on a (sub)-millimeter scale, resilient, and capable of absorbing relatively large quantities of moisture and contaminants from the environment. This makes carpet totally different from other floor coverings. Applicant recognized that furniture fabric has in essence the same typical properties as mentioned here-above, i.e being non-homogenous on a (sub)-millimeter scale, resilient, and capable of absorbing relatively large quantities of moisture and contaminants from the environment.

A similar system has been disclosed in US 2 966 790 A.

US 5,563,329 discloses a method of testing floor coverings by simulating football impacts that can be distributed over the surface of the floor covering in various patterns.

It is an object of the present invention to devise a method, the application of which can provide an adequate prediction of the suitability of a certain cleaning head and corresponding cleaning process for cleaning a specific type of fabric. A cleaning device can for example be recognized as "suitable" when upon prolonged application of this device important properties of the fabric, such as colour, dimension, yarn density, structure of the yarns etc. do not alter to a level that the change becomes immediately apparent upon a simple visual inspection.

To this end a method as specified in claim 1 has been devised.

In this method the sample of the fabric is firstly secured to a supporting structure, for example a steel table. Securing in this sense means that the position of the sample in essence remains the same during the impact of the cleaning operation. The dimensions of the sample may vary a little bit during the operation, for example due to shrinkage or swelling of the sample. The cleaning head is brought in operative connection with the sample, i.e. it is brought in a relation that at least mimics the relation of the cleaning head and a fabric during normal use of the cleaning head, i.e. a use as intended by the manufacturer of the cleaning head. Optionally, the fabric sample undergoes a pretreatment such as soiling before the cleaning head is brought in operative connection with this sample. Then the cleaning process is started and, preferably within a short timeframe before or after the process is started, a relative movement between the cleaning head and the sample is automatically imposed. After a certain period, the cleaning process is stopped, preferably within a short time frame before or after the relative movement stops, and a change in said property of the sample is established. Optionally, the sample is after-treated before the change is established. Such an after-treatment can be for example the drying of the sample if a cleaning method is used that is based on the use of a fluid, optionally comprising cleaning agents. Important properties of a fabric which can be examined for change as a result of the impact of the cleaning process are the colour of the fabric, the texture, the yarn density, the resilience and the tear strength. Changes in these properties may be established by visual inspection, but it is preferred that for each property a dedicated measurement apparatus is used. For each of these parameters, such measurement apparatus is commercially available. Depending on the specific use of the fabric, other properties then the ones mentioned here-above may be equally or even more important. It is an important advantage of the present method that when establishing the impact of the cleaning head and corresponding cleaning process on the properties of the fabric itself, at the same time the effectiveness of the cleaning process to remove dirt or other extraneous material from the fabric, can also be tested.
It is noted that a very important feature of the present invention is that the relative movement between the cleaning head and the sample is predetermined. This means that the position that the sample has with respect to the cleaning head is known beforehand at each moment during a specified time period. Thus, not only the duration of the contact between the sample and the cleaning head while performing the cleaning operation is determined beforehand, but also the relative position of the sample with respect to the cleaning head during this operation is known before it actually starts. This provides a good, unambiguous control of the impact of the cleaning head on the sample of the fabric. Another important feature in this respect is the fact that the movement is imposed automatically, i.e. essentially without human intervention. Due to these features, the overall reproducibility of the method meets high quality standards. Yet another important feature of the method according to the present invention is that the relative movement between the sample and the cleaning head comprises a rotation as well as a translation. Without being bound to theory, it is believed that due to the non-homogenous nature of fabric, the provision of both these types of movement is essential for obtaining a good predictability of the impact of the cleaning head on the fabric in real-life, e.g. when cleaning the fabric periodically during its lifetime. It is noted that the method according to the invention is not restricted to specific types of cleaning heads. For example, simple cleaners using only a rotating brush, as well as more sophisticated cleaners using rotating elements, vacuum, as well as fluids containing cleaning agents can be used for testing purposes. Thus, any type of cleaning head, such as a head of a regular vacuum cleaner, a head of a simple brush (e.g. the brush itself), a head as simple as a sponge, a head provided with wiper cloth, a wiper pad, a head of a shampoo cleaner such as the Castex Power Eagle 700, heads of blowers, extractors such as the Rotovac (available from Lamers equipment in Zelhem, The Netherlands), pressure washers, powder cleaners, pad cleaners, industrial wipers or just any other type of cleaning head that is devised to clean fabrics, in particular carpet or furniture fabric can be used in the present method.

In an embodiment the relative movement is obtained by imposing a movement on the sample and optionally imposing a movement on the cleaning head. This way the movement can be imposed very conveniently and with great accuracy.

In a further embodiment the movement of the sample comprises a translation in a first direction and a rotation, and the optional movement of the cleaning head comprises a translation in a second direction transversal to the first direction. It appears that this way, in particular when the optional movement of the cleaning head is imposed, all necessary degrees of freedom to optimally mimic the impact of the cleaning head on a fabric during normal real-life use of a cleaning device having such a cleaning head are provided.

In another embodiment the step of operatively connecting the cleaning head with the sample involves the provision of a predetermined pressure between the cleaning head and the sample. The predetermined pressure further improves the reproducibility of the method. In an embodiment the pressure is established by pneumatically or hydraulically forcing the cleaning head against the sample. This way a convenient and reliable way of providing a predetermined pressure is obtained.

In yet another embodiment of the present method environmental conditions, in particular temperature and relative humidity of the air surrounding the sample, are controlled during testing, for example by using a regular air conditioning apparatus as commercially available, to meet predetermined values. It appears that these conditions may influence the impact of the cleaning head on the fabric, and therefore, controlling these parameters further increases the reproducibility of the method as well as the predictability of change of fabric properties during real-life use of such a cleaning head.

The invention also pertains to an apparatus for use in a method as described before, the apparatus having the features specified in claim 7.

In an embodiment, the first supporting structure can be rotated with respect to the second supporting structure and translated in a direction transversal to the direction in which the rails extend.

In another embodiment, the first supporting structure is provided with pneumatic means for securing the sample of the fabric. This way a very simple and convenient way of reliably securing the sample of the fabric can be provided. Pneumatic means, as opposed to conventional mechanic means using nails, staples, clamps or comparable prior art fastening means, have the advantage that they can be switched from a clamping state to a non-clamping state. Herewith a very convenient way for securing as well as removing the sample form its supporting structure can be obtained.

In an embodiment, the second supporting structure is vertically movable under the aid of hydraulic or pneumatic means. Such means can be easily controlled, and provide a good way to adjust the pressure between the cleaning head and the sample.

In another embodiment, the apparatus comprises a central processing unit (CPU) for controlling the predetermined relative movement, which central processing unit is constituted to use programmed instructions that represent said predetermined movement. This further aids to the automatic nature of the present apparatus, and the convenience to control the operation of it. The central processing unit may be for example a personal computer which can be loaded with a program to control each and every item of the apparatus, including the cleaning machine that is used in the test, that needs adjustments or steering during operation of the apparatus. The unit may hover also be a less centralised means, such as a system comprising different sub-processors (e.g one dedicated subsystem for each motor that is used to provide the relative movement), which are interconnected via wiring or wireless connections, possibly being connected to remote control parts, for example in a control room. The latter embodiment provides the advantage that control can be provided from a remote position which for example is useful when a cleaning process involves the use of hazardous chemicals.

In a further embodiment, the apparatus is in operative connection with a memory for storing the instructions, these instruction being representative for the cleaning process of the cleaning head, wherein the instructions are automatically read from the memory by the central processing apparatus ultimately when the cleaning head is in operative connection with the sample. In this embodiment, the memory can be loaded with instructions for controlling the apparatus, wherein the instructions are representative for the actual cleaning process as it takes place in real-life. For example, although it seems of minor importance beforehand, for a vacuum cleaner a different relative movement between the cleaning head (the vacuum brush equipped head) and a carpet will be imposed in the apparatus of the invention, when compared to the relative movement between a carpet and a high speed Burnisher such as the Brady ED 7210, or a carpet extractor such as the Windsor Commodore 20 (ED4020), because the relative movement during real-life cleaning is significantly different for these types of cleaning machines when contemplating the impact of the respective cleaning heads on the properties of the carpet. In this embodiment, when a specific type of cleaning machine is secured for testing its impact on a fabric, the corresponding instructions for imposing the relative movement between the cleaning head of the apparatus and the sample of the fabric are automatically read from the memory by the central processing apparatus, e.g. because the cleaning device comprises a RFID-tag (RFID stands for Radio Frequency Identification) that is read by a scanner which is e.g. part of the apparatus such that the cleaning device is recognised by the apparatus. Alternatively, an operator of the machine could indicate, possibly by using a keyboard or other instructing means, which cleaning device is secured and made ready for testing. Thereupon, the corresponding instructions are read by the CPU. In any event, this embodiment implies that the instructions are loaded in the memory beforehand.

In an embodiment the apparatus is provided with means for automatically detecting one or more of the following parameters of the sample of the fabric: humidity, dimensions, temperature, weight and pH. These parameters can be of significant relevance for the cleaning process, so establishing these parameters increases the reproducibility of the test method performed by using the apparatus. Temperature for example gives a good indication of abrasive wear induced by the pressure between the cleaning head and the carpet sample. Humidity, optionally in combination with weight, provides an indication for the water ab- or desorbing properties of the carpet during the cleaning process. The dimensions provide information about the stability of the carpet under different environmental conditions. The weight can be used to establish the amount of remains of the cleaning process, for example the water or detergents used. In combination with the measurement of the humidity, one can exactly establish how much detergent is taken up by the carpet. The pH can be used to obtain information about cleaning agents being left in the fabric.

The invention also pertains to the use of the method or apparatus as described herein before, for establishing a change in at least one property of a tested sample of a fabric as a result of an impact of a cleaning head constituted to execute a cleaning process involving mechanical load. Thereafter, the change or a symbol or figure or any other indication corresponding to the established change of said property, may be depicted on a fabric offered for sale, label of that fabric or any other means in connection with said fabric offered for sale, if said fabric offered for sale is in essence the same as the tested fabric sample (i.e. same materials and same structure of the fabric offered for sale as compared to the fabric used for obtaining the sample for testing). Applicant has recognised that it is very important for a reseller or other entity that sells to end-users of fabrics, in particular carpets or furniture fabric, to provide adequate information about the suitability of that fabric for cleaning it with particular cleaning devices. For example, if the test shows that a sample of a carpet treated with a Windsor Rombus Carpet Maintainer 12 shows serious colour fading after several cleaning operations, an indication for this colour fading could be put on a label of the carpet corresponding to the sample when it is offered for sale. Such an indication could for example be a symbol of the type of cleaner (or even the make and model of the cleaner itself) in combination with a symbol indicating the risk of colour fading. Another possibility could for example be to put a cross through the symbol representing the machine to indicate that it is not suitable for cleaning the carpet. Note that the indication can not only be depicted on a label which is fixed to the fabric, but also on a packaging material of the fabric, or depicted in a folder or any other means used for commercially offering the fabric, such as an information page as distributed over the internet, in particular on the World Wide Web, or a medium for carrying information about the fabric such as a CD, DVD, memory stick etc.

The invention will now be further illustrated by means of the following figures.
Figure 1 depicts a side view of an apparatus adapted for applying the method according to the invention.
Figure 2 depicts a front view of the apparatus of figure 1.
Figure 3 is a top view of the supporting surface of the apparatus of figure 1 for securing a fabric sample.
Figure 4 represents a means for securing a fabric sample to a supporting surface.
Figure 5 is a schematic drawing of a labelled carpet.

### Figure 1

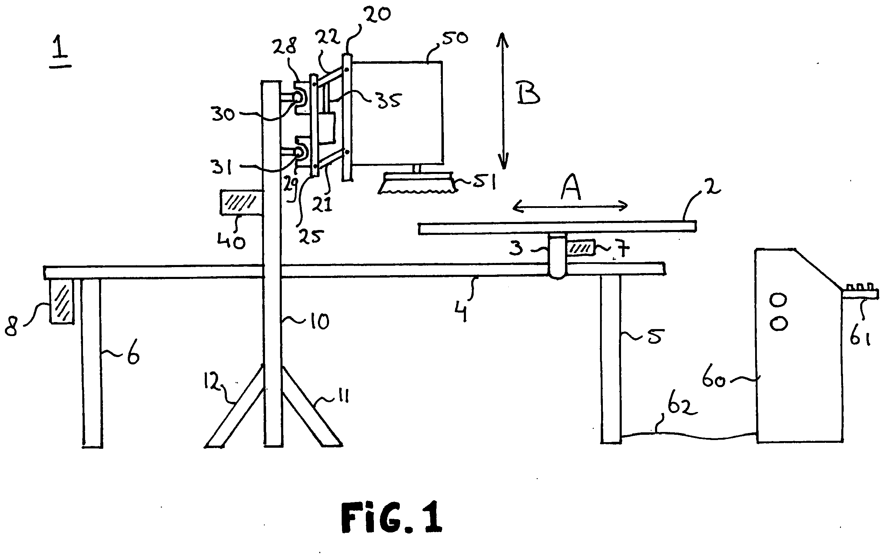

In figure 1 a side view of an apparatus adapted for applying the method according to the invention is depicted. The apparatus comprises a circular supporting surface 2 for securing one or two samples of fabrics to be tested. This surface is slidably connected to rails 4 via bracket 3. The surface 2 can be rotated by using motor 7. The rails are connected to foundation legs 5 and 6. Motor 8 can be used to slide the bracket 3, and thus surface 2, over the rails 4 in the directions indicated by capital A in figure 1. The apparatus comprises legs 10, supported by sublegs 11 and 12, to which legs rails 30 and 31 are connected. To these rails, a plate 25 is slidably connected via connecting elements 28 and 29 respectively. Supporting surface 20 is movably connected to plate 25 via arms 21 and 22. These arms allow supporting surface 20 to move in the directions indicated by capital B with respect to plate 25. This movement is controlled under the aid of hydraulic element 35. Motor 40 is used to translate element 20 along the rails 30 and 31. Secured to supporting surface 20 is cleaning device 50, which device comprises a cleaning head 51, in this case a rotating vacuum brush. The apparatus is under control of central processing apparatus 60, comprising keyboard 61 and connection cable 62. Via apparatus 60, all functions of the apparatus can be controlled. Moreover, relative movement between the surfaces 2 and 20 can be pre-programmed for simple and convenient use of the apparatus.
When using the apparatus, the cleaning head 51 is brought in operative connection with a fabric sample that is secured to surface 2. For this, cleaning device 50 is brought in a position adjacent to surface 2 by using motor 40. Surface 2 is brought underneath the cleaning head 51 by using motor 8. Then, hydraulic means 35 is used to lower cleaning device 50, until the cleaning head 51 presses the sample of the fabric with essentially the same pressure as would be the case when such a cleaning head would be used in real-life cleaning practice. Then, the cleaning process of cleaning device 50 is started and the pre-programmed relative movement between cleaning head 51 and the fabric sample is performed by automatic controlling motors 7, 8 and optionally motor 40 (depending on the type of cleaning head), by processing unit 60. During the cleaning process all movements of surfaces 2 and 20 are registered by using registration sensors (not shown). When the pro-programmed movement is stopped, the cleaning process is also stopped and the cleaning head is lifted from the fabric sample. Optionally, the fabric will be subjected to an after-treatment, such as drying of the fabric. When the fabric is ready for inspection, a change in one or more properties of the fabric can be established. In this example where a carpet sample is being tested, a change in the colour will be measured (using the LabScan XE available from HunterLab, Reston , VA, USA), as well as the dimensions (using a regular graduated ruler), the yarn density (by weighing the sample and multiplying the weight per square centimeter by the number of piled yarns per gram carpet) and the resilience (in this case by measuring the height of the carpet in rest: for many carpets the relation between the height of the carpet and the resilience is straightforward and unambiguous).

### Figure 2

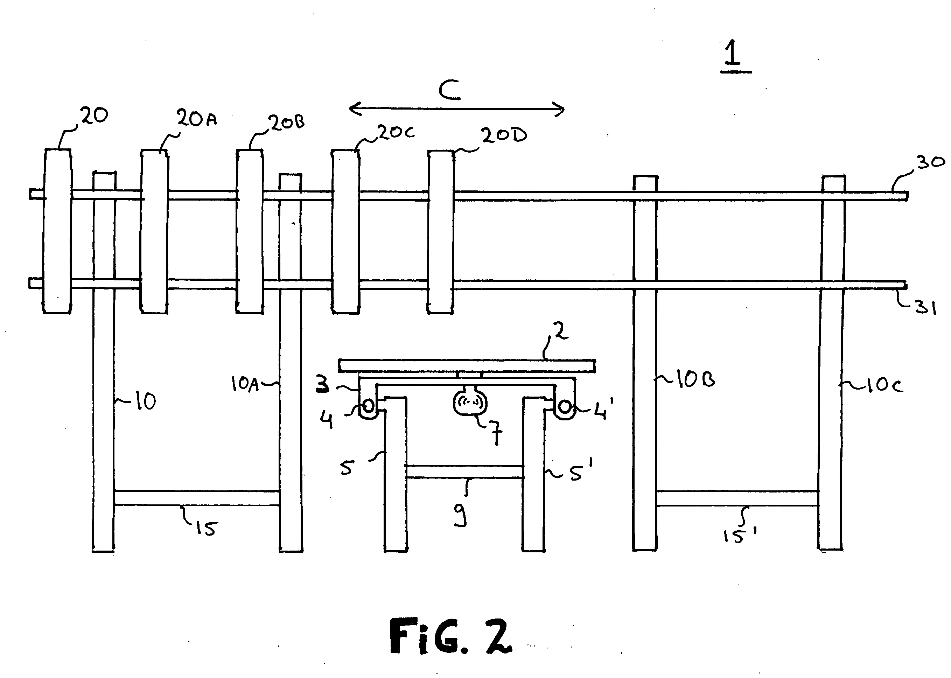

Figure 2 is a front view of the apparatus of figure 1, taken in direction from processing apparatus 60 to foundation leg 10, parallel to rails 4 (and 4'). The processing apparatus 60 itself is not shown for matters of clarity. As can be seen, element 2 is ultimately supported by two legs 5 and 5', which are interconnected by beam 9. The apparatus comprises for legs 10, namely paired legs 10 and 10A, as well as 10B and 10C, interconnected via beams 15 and 15' respectively. These legs carry elongated rails 30 and 31, which on their turn slidably support supporting elements 20, 20A, 20B, 20C and 20 D. By using motor 40 (not shown, see figure 1), each of the elements 20 can be translated in the directions indicated by capital C. By using these multiple elements 20, multiple cleaning devices can be attached to the apparatus. This provides easier testing of cleaning processes involving multiple cleaning devices, and allows more time to be used for testing.

### Figure 3

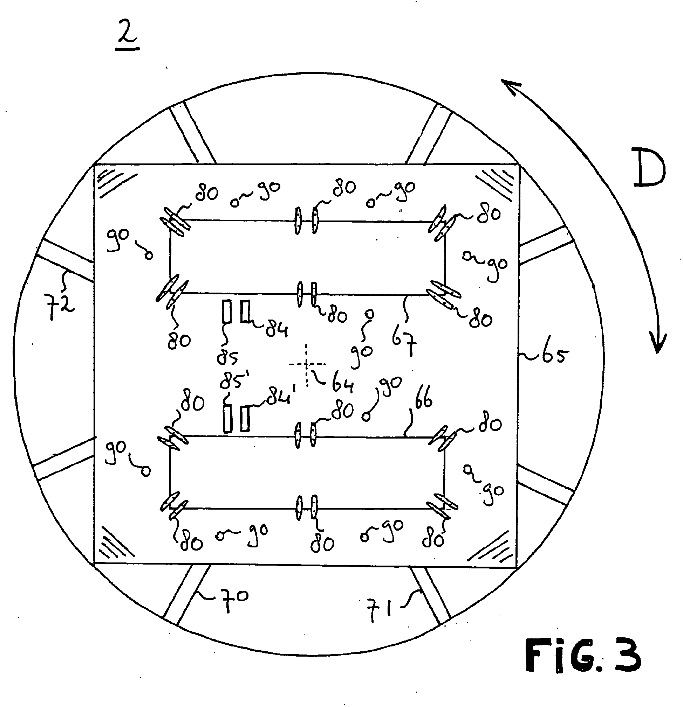

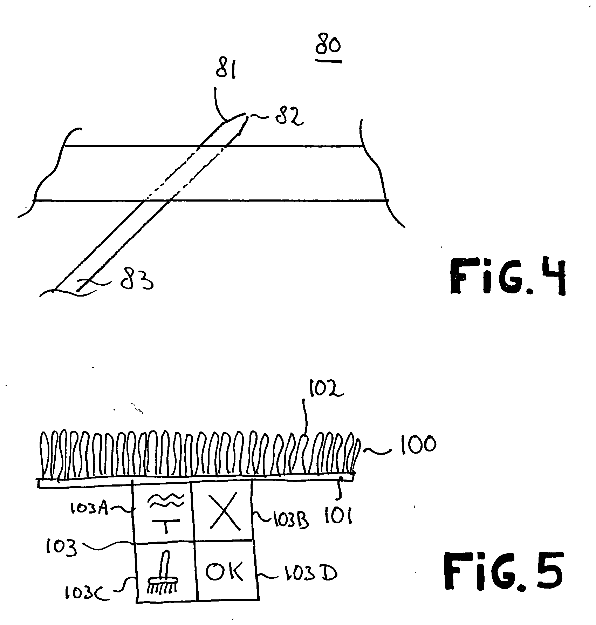

Figure 3 is a top view of the supporting surface of the apparatus of figure 1 for securing a fabric sample. The surface comprises beams 70, 71 and 72 for carrying rectangular plate 65. The plate can rotate around its pivot point 64 in the directions D as indicated. Plate 65 is provided with two areas 66 and 67 for securing samples of fabrics. For securing such a sample, the plate is provided with slots 80, wherein pneumatic means are provided (not shown here, see figure 4). These slots are provided along the boundary of areas 66 and 67. Adjacent each of these areas sensors 84 and 85 are provided for measuring the humidity (sensors 84 and 84') and temperature (sensors 85 and 85') of the fabric samples. These parameters are established not only before and after testing but also during testing of the impact of a cleaning head on the sample of a fabric, and each separate measurement is stored in a memory of element 60. This way, the cleaning history during a test (together with the registered movements) can be examined for research purposes, e.g. for optimizing test conditions. Sensors 90 are also provided, these are sensors for measuring the dimensions of the fabric, before, during and after testing. These values can also be stored in a memory of element 60.

### Figure 4

Figure 4 is a side view of a slot 80 as shown in figure 3. Needle 82 runs trough the slot and ends at the upper surface in nozzle 82. At the other side, indicate by number 83, gas can be provided at a pressure sufficient for stretching the fabric sample when place over the needles 81. This stretching provides for a tight securing of a sample on element 2. Moreover, in particular for carpet samples, it resembles the forces normally induced on a carpet when stretched for securing it to a floor.

### Figure 5

Figure 5 is a schematic drawing of a labelled carpet. Carpet 100 in this case is provided with piled yarns 102 secured to a backing 101. Label 103 is secured to the same backing. In section 103A of label 103 an icon is shown representing a cleaning head using water and heat to clean the carpet. In section 103B of the same label, the cross indicates that such a cleaning head and accompanying method is not suitable for this carpet. Such an indication can be provided when it is established, using the method according to the invention, that this carpet will undergo a significant change in one or more important properties when such a cleaning method is applied to this carpet. Section 103C of the label shows a brush, which in this case represents any brush type cleaning head, including vacuum brushes of a regular hoover. The "OK" sign in section 103D indicates that this type of cleaning head is suitable for this carpet. This type of information is particularly important for an end-user who is considering to purchase a carpet of this type.

## Claims

1. Method for testing an impact of a cleaning head (51) constituted to execute a cleaning process involving mechanical load, on a property of a fabric (100), comprising:
- securing a sample of the fabric (100) on a supporting structure (2),
- operatively connecting the cleaning head (51) with the sample,
- starting the cleaning process of the cleaning head (51),
- automatically imposing a predetermined relative movement of the sample between the cleaning head (51) and the sample while the cleaning head (51) and the sample are operatively connected,
- stopping the cleaning process,
- and establishing a change in said property of the sample,
**characterized in that** the relative movement comprises a rotation and a translation.

2. Method according to claim 1, **characterised in that** the relative movement is obtained by imposing a movement on the sample and optionally imposing a movement on the cleaning head (51).

3. Method according to claim 2, **characterised in that** the movement of the sample comprises a translation in a first direction and a rotation, and that the optional movement of the cleaning head (51) comprises a translation in a second direction transversal to the first direction.

4. Method according to any of the preceding claims, **characterised in that** the step of operatively connecting the cleaning head (51) with the sample involves the provision of a predetermined pressure between the cleaning head (51) and the sample.

5. Method according to claim 5, **characterised in that** the pressure is established by pneumatically or hydraulically forcing the cleaning head (51) against the sample.

6. Method according to any of the preceding claims, **characterised in that** environmental conditions, in particular temperature and relative humidity of the air surrounding the sample, during testing are controlled to meet predetermined values.

7. Apparatus (1) for use in a method for testing the impact of a cleaning head (51) constituted to execute a cleaning process involving mechanical load on a property of a fabric (100), comprising:
- a first supporting structure (2) for securing a sample of a fabric,
- a second supporting structure (20) for securing the cleaning head in operative connection with the sample, and
- means (60, 7, 8, 40) for automatically imposing a predetermined relative movement of the sample between the cleaning head (51) and the sample while the cleaning head (51) and the sample are operatively connected,
**characterized in that** the relative movement comprises a rotation and a translation.

8. Apparatus (1) according to claim 7, **characterised in that** it comprises elongated rails (30, 31) that carry multiple second supporting structures (20, 20A, 20B, 20C, 20D), each of them being movable along the rails (30, 31) in order to guide them to a position adjacent to the first supporting structure (2).

9. Apparatus (1) according to claim 8, **characterised in that** the first supporting structure (2) can be rotated with respect to the second supporting structure (20) and translated in a direction transversal to the direction in which the rails (30, 31) extend.

10. Apparatus (1) according to any of the claims 7 to 9, **characterised in that** the first supporting structure (2) is provided with pneumatic means (80, 81, 82, 83) for securing the sample of the fabric.

11. Apparatus (1) according any of the claims 7 to 10, **characterised in that** the second supporting structure (20) is vertically movable under the aid of hydraulic or pneumatic means (35).

12. Apparatus (1) according to claim any of the claims 7 or 11, **characterised in that** the apparatus (1) comprises a central processing unit (60) for controlling the predetermined relative movement, which central processing unit is constituted to use programmed instructions that represent said predetermined movement.

13. Apparatus (1) according to claim 12, wherein the apparatus (1) is in operative connection with a memory for storing the instructions, these instruction being representative for the cleaning process of the cleaning head (51), **characterised in that** the instructions are automatically read from the memory by the central processing apparatus (60) ultimately when the cleaning head (51) is in operative connection with the sample.

14. Apparatus (1) according to any of the claims 7 to 13, **characterised in that** it is provided with means (84, 85, 90) for automatically detecting one or more of the following parameters of the sample of the fabric: humidity, dimensions, temperature, weight and pH.

15. Use of a method according to any of the claims 1 to 6 or an apparatus (1) according to any of the claims 7 to 14 for establishing a change in at least one property of a tested sample of a fabric (100) as a result of an impact of a cleaning head (51) constituted to execute a cleaning process involving mechanical load.

## Patentansprüche

1. Verfahren zum Prüfen der Wirkung eines Reinigungskopfes (51), der dazu ausgebildet ist, einen Reinigungsvorgang mit mechanischer Einwirkung auszuführen, auf eine Eigenschaft eines Stoffes (100), mit:
- Befestigen einer Probe des Stoffes (100) auf einer Tragstruktur (2),
- funktionsmäßigem Verbinden des Reinigungskopfes (51) mit der Probe,
- Starten des Reinigungsprozesses des Reinigungskopfes (51),
- automatischem Erzeugen einer vorbestimmten Relativbewegung der Probe zwischen dem Reinigungskopf (51) und der Probe, während der Reinigungskopf (51) und die Probe funktionsmäßig verbunden sind,
- Anhalten des Reinigungsprozesses,
- und Feststellen einer Änderung in der genannten Eigenschaft des Stoffes,
**dadurch gekennzeichnet, dass** die Relativbewegung eine Rotation und eine Translation umfasst.

2. Verfahren nach Anspruch 1, **dadurch gekennzeichnet, dass** die Relativbewegung erhalten wird durch Erzeugen einer Bewegung der Probe und optional Erzeugen einer Bewegung des Reinigungskopfes (51).

3. Verfahren nach Anspruch 2, **dadurch gekennzeichnet**, das die Bewegung der Probe eine Translation in einer ersten Richtung und eine Rotation umfasst und dass die optionale Bewegung des Reinigungskopfes (51) eine Translation in einer zweiten Richtung quer zu der ersten Richtung umfasst.

4. Verfahren nach einem der vorstehenden Ansprüche, **dadurch gekennzeichnet, dass** der Schritt der funktionsmäßigen Verbindung des Reinigungskopfes (51) mit der Probe das Ausüben eines vorbestimmten Druckes zwischen dem Reinigungskopf (51) und der Probe einschließt.

5. Verfahren nach Anspruch 5, **dadurch gekennzeichnet, dass** der Druck erzeugt wird durch pneumatisches oder hydraulisches Andrücken des Reinigungskopfes (51) gegen die Probe.

6. Verfahren nach einem der vorstehenden Ansprüche, **dadurch gekennzeichnet, dass** Umgebungsbedingungen, insbesondere Temperatur und relative Feuchte der die Probe umgebende Luft, während der Prüfung kontrolliert werden, um vorbestimmte Werte einzuhalten.

7. Vorrichtung (1) zur Verwendung in einem Verfahren zum Prüfen der Wirkung eines Reinigungskopfes (51), der dazu ausgebildet ist, einen Reinigungsprozess mit mechanischer Einwirkung auszuführen, auf eine Eigenschaft eines Stoffes (100), mit:
- einer ersten Tragstruktur (2) zum Befestigen einer Probe des Stoffes,
- einer zweiten Tragstruktur (20) zum Befestigen des Reinigungskopfes in funktionsmäßiger Verbindung mit der Probe, und
- Mitteln (60, 7, 8, 40) zum automatischen Erzeugen einer vorbestimmten Relativbewegung der Probe zwischen dem Reinigungskopf (51) und der Probe, während der Reinigungskopf (51) und die Probe funktionsmäßig verbunden sind,
**dadurch gekennzeichnet, dass** die Relativbewegung eine Rotation und eine Translation umfasst.

8. Vorrichtung (1) nach Anspruch 7, **dadurch gekennzeichnet, dass** sie langgestreckte Schienen (30, 31) umfasst, die mehrere zweite Tragstrukturen (20, 20A, 20B, 20C, 20D) tragen, von denen jede entlang der Schienen (30, 31) beweglich ist, um sie zu einer an die erste Tragstruktur (2) angrenzenden Position zu führen.

9. Vorrichtung (1) nach Anspruch 8, **dadurch gekennzeichnet, dass** die erste Tragstruktur (2) in Bezug auf die zweite Tragstruktur (20) gedreht werden kann und in einer Richtung quer zu der Richtung, in der sich die Schienen (30, 31) erstrecken, in Translation bewegt werden kann.

10. Vorrichtung (1) nach einem der Ansprüche 7 bis 9, **dadurch gekennzeichnet, dass** die erste Tragstruktur (2) pneumatische Mittel (80, 81, 82, 83) zum Befestigen der Probe des Stoffes aufweist.

11. Vorrichtung (1) nach einem der Ansprüche 7 bis 10, **dadurch gekennzeichnet, dass** die zweite Tragstruktur (20) mit Hilfe von hydraulischen oder pneumatischen Mitteln (35) vertikal beweglich ist.

12. Vorrichtung (1) nach einem der Ansprüche 7 oder 11, **dadurch gekennzeichnet, dass** die Vorrichtung (1) eine zentrale Prozessoreinheit (60) zur Steuerung der vorbestimmten Relativbewegung aufweist, welche zentrale Prozessoreinheit dazu ausgebildet ist, programmierte Instruktionen zu verwenden, die die vorbestimmte Bewegung repräsentieren.

13. Vorrichtung (1) nach Anspruch 12, bei der die Vorrichtung (1) in funktionsmäßiger Verbindung mit einem Speicher zur Speicherung der Instruktionen steht, wobei die Instruktionen für den Reinigungsprozess des Reinigungskopfes (51) repräsentativ sind, **dadurch gekennzeichnet, dass** die Instruktionen von der zentralen Prozessoreinheit (60) automatisch ultimativ aus dem Speicher gelesen werden, wenn der Reinigungskopf (51) in funktionsmäßiger Verbindung mit der Probe steht.

14. Vorrichtung (1) nach einem der Ansprüche 7 bis 13, **dadurch gekennzeichnet, dass** sie mit Mitteln (84, 85, 90) zum automatischen Detektieren eines oder mehrerer der folgenden Parameter der Probe des Stoffes ausgestattet ist: Feuchtigkeit, Abmessungen, Temperatur, Gewicht und pH.

15. Verwendung eines Verfahrens nach einem der Ansprüche 1 bis 6 oder einer Vorrichtung (1) nach einem der Ansprüche 7 bis 14 zum Feststellen einer Änderung in wenigstens einer Eigenschaft einer getesteten Probe eines Stoffes (100) als Ergebnis einer Einwirkung eines Reinigungskopfes (51), der dazu ausgebildet ist, einen Reinigungsprozess mit mechanischer Beanspruchung auszuführen.

## Revendications

1. Procédé pour tester un impact d'une tête de nettoyage (51) formée de manière à exécuter un processus de nettoyage impliquant une charge mécanique, sur une propriété d'un tissu (100), comportant les étapes consistant à :
- fixer un échantillon du tissu (100) sur une structure de support (2),
- relier la tête de nettoyage (51) à l'échantillon de manière opérationnelle,
- lancer le processus de nettoyage de la tête de nettoyage (51),
- imposer automatiquement un mouvement relatif prédéterminé de l'échantillon entre la tête de nettoyage (51) et l'échantillon lorsque la tête de nettoyage (51) et l'échantillon sont reliés de manière opérationnelle,
- arrêter le processus de nettoyage,
- et établir un changement de ladite propriété de l'échantillon,
**caractérisé en ce que** le mouvement relatif comporte une rotation et une translation.

2. Procédé selon la revendication 1, **caractérisé en ce que** le mouvement relatif est obtenu en imposant un mouvement à l'échantillon et en imposant facultativement un mouvement à la tête de nettoyage (51).

3. Procédé selon la revendication 2, **caractérisé en ce que** le mouvement de l'échantillon comporte une translation dans une première direction et une rotation, et que le mouvement facultatif de la tête de nettoyage (51) comporte une translation dans une seconde direction transversale à la première direction.

4. Procédé selon l'une quelconque des revendications précédentes, **caractérisé en ce que** l'étape consistant à relier la tête de nettoyage (51) à l'échantillon de manière opérationnelle implique la fourniture d'une pression prédéterminée entre la tête de nettoyage (51) et l'échantillon.

5. Procédé selon la revendication 4, **caractérisé en ce que** la pression est établie en forçant pneumatiquement ou hydrauliquement la tête de nettoyage (51) contre l'échantillon.

6. Procédé selon l'une quelconque des revendications précédentes, **caractérisé en ce que** les conditions environnementales, en particulier la température et l'humidité relative de l'air entourant l'échantillon, pendant le test sont contrôlées pour satisfaire à des valeurs prédéterminées.

7. Appareil (1) à utiliser dans un procédé pour tester l'impact d'une tête de nettoyage (51) formée de manière à exécuter un processus de nettoyage impliquant une charge mécanique sur une propriété d'un tissu (100), comportant :
- une première structure de support (2) pour fixer un échantillon d'un tissu,
- une seconde structure de support (20) pour fixer la tête de nettoyage en liaison opérationnelle avec l'échantillon, et
- des moyens (60, 7, 8, 40) pour imposer automatiquement un mouvement relatif prédéterminé de l'échantillon entre la tête de nettoyage (51) et l'échantillon lorsque la tête de nettoyage (51) et l'échantillon sont reliés de manière opérationnelle,
**caractérisé en ce que** le mouvement relatif comporte une rotation et une translation.

8. Appareil (1) selon la revendication 7, **caractérisé en ce qu'**il comporte des rails allongés (30, 31) qui supportent de multiples secondes structures de support (20, 20A, 20B, 20C, 20D), chacune d'elle étant mobile le long des rails (30, 31) afin de les guider jusqu'à une position adjacente à la première structure de support (2).

9. Appareil (1) selon la revendication 8, **caractérisé en ce que** la première structure de support (2) peut tourner par rapport à la seconde structure de support (20) et être déplacée en translation dans une direction transversale à la direction dans laquelle s'étendent les rails (30, 31).

10. Appareil (1) selon l'une quelconque des revendications 7 à 9, **caractérisé en ce que** la première structure de support (2) est pourvue de moyens pneumatiques (80, 81, 82, 83) pour fixer l'échantillon du tissu.

11. Appareil (1) selon l'une quelconque des revendications 7 à 10, **caractérisé en ce que** la seconde structure de support (20) est verticalement mobile à l'aide de moyens hydrauliques ou pneumatiques (35).

12. Appareil (1) selon l'une quelconque des revendications 7 ou 11, **caractérisé en ce que** l'appareil (1) comporte une unité centrale de traitement (60) pour commander le mouvement relatif prédéterminé, laquelle unité centrale de traitement est formée de manière à utiliser des instructions programmées qui représentent ledit mouvement prédéterminé.

13. Appareil (1) selon la revendication 12, dans lequel l'appareil (1) est en liaison opérationnelle avec une mémoire pour stocker des instructions, ces instructions étant représentatives du processus de nettoyage de la tête de nettoyage (51), **caractérisé en ce que** les instructions sont automatiquement lues à partir de la mémoire par l'unité centrale de traitement (60) finalement lorsque la tête de nettoyage (51) est en liaison opérationnelle avec l'échantillon.

14. Appareil (1) selon l'une quelconque des revendications 7 à 13, **caractérisé en ce qu'**il est pourvu de moyens (84, 85, 90) pour détecter automatiquement un ou plusieurs des paramètres suivants de l'échantillon du tissu : humilité, dimensions, température, poids et pH.

15. Utilisation d'un procédé selon l'une quelconque des revendications 1 à 6 ou d'un appareil (1) selon l'une quelconque des revendications 7 à 14 pour établir un changement d'au moins une propriété d'un échantillon testé d'un tissu (100) en résultat d'un impact d'une tête de nettoyage (51) formée de manière à exécuter un processus de nettoyage impliquant une charge mécanique.
